# NEW EUROPEAN PATENT SPECIFICATION

(11) **EP 1 399 200 B2**
(45) Date of publication and mention of the opposition decision: **02.07.2014**
(45) Mention of the grant of the patent: 31.08.2005
(21) Application number: 02734754.1
(22) Date of filing: 11.06.2002
(51) Int. Cl.: A61L 27/48, A61L 27/18, A61L 27/50, A61L 33/00, A61F 2/06, A61M 39/04, A61M 39/10

(54) **COMPOSITE ePTFE/TEXTILE PROSTHESIS**
KOMPOSIT EPTFE/TEXTIL PROTHESE
PROTHESE COMPOSITE EPTFE/TEXTILE

(30) Priority: 11.06.2001 US 297401 P
(43) Date of publication of application: 24.03.2004
(73) Proprietor: Boston Scientific Limited, St. Michael, Barbados, West Indies (BB)
(72) Inventor: SOWINSKI, Krzysztof, Wallington, NJ 07057 (US); RAKOS, Ronald, Neshanic Station, NJ 08853 (US); DONG, Jerry, Oakland, NJ 07436 (US); KUJAWSKI, Denis, Warwick, NY 10990 (US)
(74) Representative: Hermann, Gerhard
(86) International application number: PCT/US2002/018303
(87) International publication number: WO 2002/100454

(56) References cited:
- WO-A-00/47271
- WO-A-95/22359
- WO-A1-90/14054
- WO-A1-94/19029
- WO-A1-99/32051
- WO-A1-02/100454
- US-A- 3 142 067
- US-A- 3 953 566
- US-A- 3 962 153
- US-A- 4 619 641
- US-A- 5 163 951
- US-A- 5 527 352
- US-A- 5 749 880
- US-A- 5 824 054
- US-A- 5 976 192
- US-A- 6 001 056
- US-A- 6 036 724
- US-A- 6 080 198
- SCHANZER H. ET AL.: 'A Self-sealing Dialysis Prosthesis' ANN. SURG. vol. 204, no. 5, November 1986,

## Description

### CROSS-REFERENCE TO RELATED APPLICATION

The present invention claims priority to U.S. Provisional Patent Application No. 60/279,401, filed June 11, 2001. The present application is being concurrently filed with Attorney Docket No. 498-270.

### FIELD OF THE INVENTION:

The present invention relates generally to an implantable prosthesis. More particularly, the present invention relates to a composite multilayer implantable structure having a textile layer, an expanded polytetrafluoroethylene layer (ePTFE) and an elastomeric bonding agent layer within the ePTFE porous layer, which joins the textile and ePTFE layer to form an integral structure.

### BACKGROUND OF THE INVENTION:

Implantable prostheses are commonly used in medical applications. One of the more common prosthetic structures is a tubular prosthesis which may be used as a vascular graft to replace or repair damaged or diseased blood vessel. To maximize the effectiveness of such a prosthesis, it should be designed with characteristics which closely resemble that of the natural body lumen which it is repairing or replacing.

One form of a conventional tubular prosthesis specifically used for vascular grafts includes a textile tubular structure formed by weaving, knitting, braiding or any non-woven textile technique processing synthetic fibers into a tubular configuration. Tubular textile structures have the advantage of being naturally porous which allows desired tissue ingrowth and assimilation into the body. This porosity, which allows for ingrowth of surrounding tissue, must be balanced with fluid tightness so as to minimize leakage during the initial implantation stage.

Attempts to control the porosity of the graft while providing a sufficient fluid barrier have focused on increasing the thickness of the textile structure, providing a tighter stitch construction and incorporating features such as velours to the graft structure. Further, most textile grafts require the application of a biodegradable natural coating, such as collagen or gelatin in order to render the graft blood tight. While grafts formed in this manner overcome certain disadvantages inherent in attempts to balance porosity and fluid tightness, these textile prostheses may exhibit certain undesirable characteristics. These characteristics may include an undesirable increase in the thickness of the tubular structure, which makes implantation more difficult. These textile tubes may also be subject to kinking, bending, twisting or collapsing during handling. Moreover, application of a coating may render the grafts less desirable to handle from a tactility point of view.

It is also well known to form a prosthesis, especially a tubular graft, from polymers such as polytetrafluoroethylene (PTFE). A tubular graft may be formed by stretching and expanding PTFE into a structure referred to as expanded polytetrafluoroethylene (ePTFE). Tubes formed of ePTFE exhibit certain beneficial properties as compared with textile prostheses. The expanded PTFE tube has a unique structure defined by nodes interconnected by fibrils. The node and fibril structure defines micropores which facilitate a desired degree of tissue ingrowth while remaining substantially fluid-tight. Tubes of ePTFE may be formed to be exceptionally thin and yet exhibit the requisite strength necessary to serve in the repair or replacement of a body lumen. The thinness of the ePTFE tube facilitates ease of implantation and deployment with minimal adverse impact on the body.

While exhibiting certain superior attributes, ePTFE tubes are not without certain disadvantages. Grafts formed of ePTFE tend to be relatively non-compliant as compared with textile grafts and natural vessels. Further, while exhibiting a high degree of tensile strength, ePTFE grafts are susceptible to tearing. Additionally, ePTFE grafts lack the suture compliance of coated textile grafts. This may cause undesirable bleeding at the suture hole. Thus, the ePTFE grafts lack many of the advantageous properties of certain textile grafts.

It is also known that it is extremely difficult to join PTFE and ePTFE to other materials via adhesives or bonding agents due to its chemically inert and non-wetting character. Wetting of the surface by the adhesive is necessary to achieve adhesive bonding, and PTFE and ePTFE are extremely difficult to wet without destroying the chemical properties of the polymer. Thus, heretofore, attempts to bond ePTFE to other dissimilar materials such as textiles, have been difficult.

It is apparent that conventional textile prostheses as well as ePTFE prostheses have acknowledged advantages and disadvantages. Neither of the conventional prosthetic materials exhibits fully all of the benefits desirable for use as a vascular prosthesis.

It is therefore desirable to provide an implantable prosthesis, preferably in the form of a tubular vascular prosthesis, which achieves many of the above-stated benefits without the resultant disadvantages associated therewith. It is also desirable to provide an implantable multi-layered patch which also achieves the above-stated benefits without the disadvantages of similar conventional products.

### SUMMARY OF THE INVENTION:

The present invention provides a composite multi-layered implantable prosthetic structure which may be used in various applications, especially vascular applications. The implantable structure of the present invention may include an ePTFE-lined textile graft, an ePTFE graft, covered with a textile covering, or a vascular patch including a textile surface and an opposed ePTFE surface. Moreover, additional ePTFE and/or textile layers may be combined with any of these embodiments.

The composite multi-layered implantable structure of the present invention includes a first layer formed of a textile material and a second layer formed of expanded polytetrafluoroethylene (ePTFE) having a porous microstructure defined by nodes interconnected by fibrils. An elastomeric bonding agent is applied to either the first or the second layer and disposed within the pores of the microstructure for securing the first layer to the second layer.

The bonding agent may be selected from a group of materials including biocompatible elastomeric materials such as urethanes, silicones, isobutylene/styrene copolymers, block polymers and combinations thereof.

The tubular composite grafts of the present invention may also be formed from appropriately layered sheets which can then be overlapped to form tubular structures. Bifurcated, tapered conical and stepped-diameter tubular structures may also be formed from the present invention.

The first layer may be formed of various textile structures including knits, weaves, stretch knits, braids, any non-woven textile processing techniques, and combinations thereof. Various biocompatible polymeric materials may be used to form the textile structures, including polyethylene terephthalate (PET), naphthalene dicarboxylate derivatives such as polyethylene naphthalate, polybutylene naphthalate, polytrimethylene naphthalate, trimethylenediol naphthalate, ePTFE, natural silk, polyethylene and polypropylene, among others. PET is a particularly desirable material for forming the textile layer.

The bonding agent may be applied in a number of different forms to either the first or second layer. Preferably, the bonding agent is applied in solution to one surface of the ePTFE layer, preferably by spray coating. The textile layer is then placed in contact with the coated surface of the ePTFE layer. The bonding agent may also be in the form of a solid tubular structure. The bonding agent may also be applied in powder form, and may also be applied and activated by thermal and/or chemical processing well known in the art.

The present invention more specifically provides an ePTFE-lined textile graft. The lined textile graft includes a tubular textile substrate bonded using a biocompatible elastomeric material to a tubular liner of ePTFE. A coating of an elastomeric bonding agent may be applied to the surface of the ePTFE liner so that the bonding agent is present in the micropores thereof. The coated liner is then secured to the tubular textile structure via the elastomeric binding agent. The liner and textile graft can each be made very thin and still maintain the advantages of both types of materials.

The present invention further provides a textile-covered ePTFE graft. The tubular ePTFE graft structure includes micropores defined by nodes interconnected by fibrils. A coating of an elastomeric bonding agent is applied to the surface of the ePTFE tubular structure with the bonding agent being resident within the microporous structure thereof. A tubular textile structure is applied to the coated surface of the ePTFE tubular structure and secured thereto by the elastomeric bonding agent.

Additionally, the present invention provides an implantable patch which may be used to cover an incision made in a blood vessel, or otherwise support or repair a soft tissue body part, such as a vascular wall. The patch of the present invention includes an elongate ePTFE substrate being positioned as the interior surface of a vascular wall. The opposed surface is coated with a bonding agent, such that the bonding agent resides within the microporous structure of the ePTFE substrate. A planar textile substrate is positioned over the coated surface of the ePTFE substrate so as to form a composite multi-layered implantable structure.

The composite multi-layered implantable structures of the present invention are designed to take advantage of the inherent beneficial properties of the materials forming each of the layers. The textile layer provides for enhanced tissue ingrowth, high suture retention strength and longitudinal compliance for ease of implantation. The ePTFE layer provides the beneficial properties of sealing the textile layer without need for coating the textile layer with a sealant such as collagen. The sealing properties of the ePTFE layer allow the wall thickness of the textile layer to be minimized. Further, the ePTFE layer exhibits enhanced thrombo-resistance upon implantation. Moreover, the elastomeric bonding agent not only provides for an integral composite structure, but also may add further puncture-sealing characteristics to the final prosthesis.

Various additives such as drugs, growth-factors, anti-microbial, anti-thrombogenic agents and the like may also be employed.

### BRIEF DESCRIPTION OF THE DRAWINGS:

Figure 1 shows a schematic cross-section, a portion of a composite multi-layered implantable structure of the present invention.
Figures 2 and 3 show an ePTFE-lined textile grafts of the present invention.
Figures 4, 5 and 6 show an ePTFE graft with a textile coating of the present invention.
Figures 7-10 show the ePTFE graft with a textile coating of Figure 4 with an external coil applied thereto.
Figures 11-13 show a composite ePTFE textile vascular patch of the present invention.

### DETAILED DESCRIPTION OF THE PREFERRED EMBODIMENT:

The present invention provides a composite implantable prosthesis, desirably a vascular prosthesis including a layer of ePTFE and a layer of a textile material which are secured together by an elastomeric bonding agent. The vascular prosthesis of the present invention may include a ePTFE-lined textile vascular graft, an ePTFE vascular graft including a textile covering and a composite ePTFE/textile vascular patch.

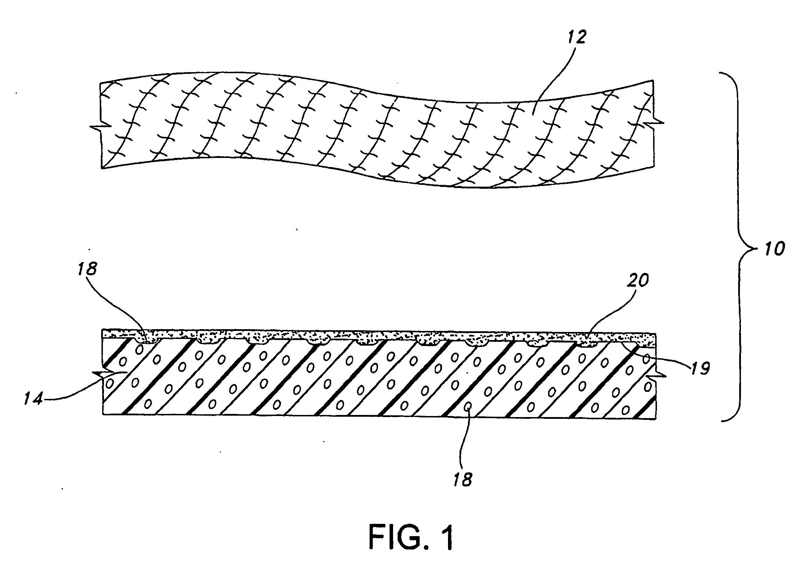

Referring to Figure 1, a schematic cross-section of a portion of a representative vascular prosthesis 10 is shown. As noted above, the prosthesis 10 may be a portion of a graft, patch or any other implantable structure.

The prosthesis 10 includes a first layer 12 which is formed of a textile material. The textile material 12 of the present invention may be formed from synthetic yarns that may be flat, shaped, twisted, textured, pre-shrunk or un-shrunk. Preferably, the yarns are made from thermoplastic materials including, but not limited to, polyesters, polypropylenes, polyethylenes, polyurethanes, polynaphthalenes, polytetrafluoroethylenes and the like. The yarns may be of the multifilament, monofilament or spun types. In most vascular applications, multifilaments are preferred due to the increase in flexibility. Where enhanced crush resistance is desired, the use of monofilaments have been found to be effective. As is well known, the type and denier of the yarn chosen are selected in a manner which forms a pliable soft tissue prosthesis and, more particularly, a vascular structure have desirable properties.

The prosthesis 10 further includes a second layer 14 formed of expanded polytetrafluoroethylene (ePTFE). The ePTFE layer 14 may be produced from the expansion of PTFE formed in a paste extrusion process. The PTFE extrusion may be expanded and sintered in a manner well known in the art to form ePTFE having a microporous structure defined by nodes interconnected by elongate fibrils. The distance between the nodes, referred to as the internodal distance (IND), may be varied by the parameters employed during the expansion and sintering process. The resulting process of expansion and sintering yields pores 18 within the structure of the ePTFE layer. The size of the pores are defined by the IND of the ePTFE layer.

The composite prosthesis 10 of the present invention further includes a bonding agent 20 applied to one surface 19 of ePTFE layer 18. The bonding agent 20 is preferably applied in solution by a spray coating process. However, other processes may be employed to apply the bonding agent.

In the present invention, the bonding agent may include various biocompatible, elastomeric bonding agents such as urethanes, styrene/isobutylene/styrene block copolymers (SIBS), silicones, and combinations thereof. Other similar materials are contemplated. Most desirably, the bonding agent may include polycarbonate urethanes sold under the trade name CORETHANE®. This urethane is provided as an adhesive solution with preferably 7.5% Corethane, 2.5 W30, in dimethylacetamide (DMAc) solvent.

The term elastomeric as used herein refers to a substance having the characteristic that it tends to resume an original shape after any deformation thereto, such as stretching, expanding or compression.

The polycarbonate urethane polymers particularly useful in the present invention are more fully described in U.S. Patent Nos. 5,133,742 and 5,229,431. These polymers are particularly resistant to degradation in the body over time and exhibit exceptional resistance to cracking *in vivo*. These polymers are segmented polyurethanes which employ a combination of hard and soft segments to achieve their durability, biostability, flexibility and elastomeric properties.

The polycarbonate urethanes useful in the present invention are prepared from the reaction of an aliphatic or aromatic polycarbonate macroglycol and a diisocyanate n the presence of a chain extender. Aliphatic polycarbonate macroglycols such as polyhexane carbonate macroglycols and aromatic diisocyanates such as methylene diisocyanate are most desired due to the increased biostability, higher intramolecular bond strength, better heat stability and flex fatigue life, as compared to other materials.

The polycarbonate urethanes particularly useful in the present invention are the reaction products of a macroglycol, a diisocyanate and a chain extender.

A polycarbonate component is characterized by repeating 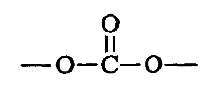 units, and a general formula for a polycarbonate macroglycol is as follows: 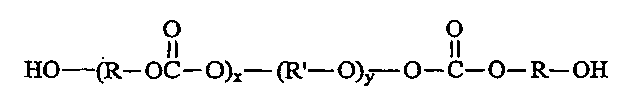 wherein x is from 2 to 35, y is 0, 1 or 2, R either is cycloaliphatic, aromatic or aliphatic having from 4 to 40 carbon atoms or is alkoxy having from 2 to 20 carbon atoms, and wherein R' has from 2 to 4 linear carbon atoms with or without additional pendant carbon groups.

Examples of typical aromatic polycarbonate macroglycols include those derived from phosgene and bisphenol A or by ester exchange between bisphenol A and diphenyl carbonate such as (4,4'-dihydroxy-diphenyl-2,2'-propane) shown below, wherein n is between 1 and 12.

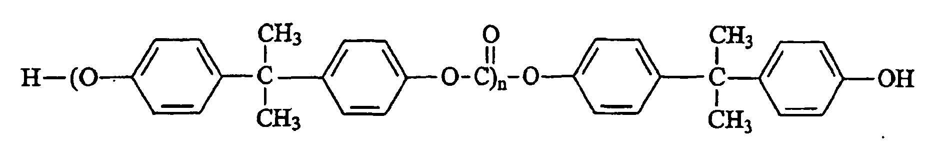

Typical aliphatic polycarbonates are formed by reacting cycloaliphatic or aliphatic diols with alkylene carbonates as shown by the general reaction below: 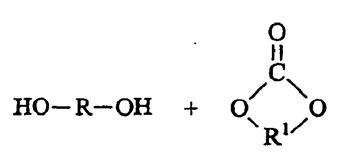 wherein R is cyclic or linear and has between 1 and 40 carbon atoms and wherein R¹ is linear and has between 1 and 4 carbon atoms.

Typical examples of aliphatic polycarbonate diols include the reaction products of 1,6-hexanediol with ethylene carbonate, 1,4-butanediol with propylene carbonate, 1,5-pentanediol with ethylene carbonate, cyclohexanedimethanol with ethylene carbonate and the like and mixtures of above such as diethyleneglycol and cyclohexanedimethanol with ethylene carbonate.

When desired, polycarbonates such as these can be copolymerized with components such as hindered polyesters, for example phthalic acid, in order to form carbonate/ester copolymer macroglycols. Copolymers formed in this manner can be entirely aliphatic, entirely aromatic, or mixed aliphatic and aromatic. The polycarbonate macroglycols typically have a molecular weight of between 200 and 4000 Daltons.

Diisocyanate reactants according to this invention have the general structure OCN-R'-NCO, wherein R' is a hydrocarbon that may include aromatic or nonaromatic structures, including aliphatic and cycloaliphatic structures. Exemplary isocyanates include the preferred methylene diisocyanate (MDI), or 4,4-methylene bisphenyl isocyanate, or 4,4'-diphenylmethane diisocyanate and hydrogenated methylene diisocyanate (HMDI). Other exemplary isocyanates include hexamethylene diisocyanate and other toluene diisocyanates such as 2,4-toluene diisocyanate and 2,6-toluene diisocyanate, 4,4' tolidine diisocyanate, m-phenylene diisocyanate, 4-chloro-1,3-phenylene diisocyanate, 4,4-tetramethylene diisocyanate, 1,6-hexamethylene diisocyanate, 1,10-decamethylene diisocyanate, 1,4-cyclohexylene diisocyanate, 4,4'-methylene bis (cyclohexylisocyanate), 1,4-isophorone diisocyanate, 3,3'-dimethyl-4,4'-diphenylmethane diisocyanate, 1,5-tetrahydronaphthalene diisocyanate, and mixtures of such diisocyanates. Also included among the isocyanates applicable to this invention are specialty isocyanates containing sulfonated groups for improved hemocompatibility and the like.

Suitable chain extenders included in this polymerization of the polycarbonate urethanes should have a functionality that is equal to or greater than two. A preferred and well-recognized chain extender is 1,4-butanediol. Generally speaking, most diols or diamines are suitable, including the ethylenediols, the propylenediols, ethylenediamine, 1,4-butanediamine methylene dianiline heteromolecules such as ethanolamine, reaction products of said diisocyanates with water and combinations of the above.

The polycarbonate urethane polymers according to the present invention should be substantially devoid of any significant ether linkages (i.e., when y is 0, 1 or 2 as represented in the general formula hereinabove for a polycarbonate macroglycol), and it is believed that ether linkages should not be present at levels in excess of impurity or side reaction concentrations. While not wishing to be bound by any specific theory, it is presently believed that ether linkages account for much of the degradation that is experienced by polymers not in accordance with the present invention due to enzymes that are typically encountered in vivo, or otherwise, attack the ether linkage via oxidation. Live cells probably catalyze degradation of polymers containing linkages. The polycarbonate urethanes useful in the present invention avoid this problem.

Because minimal quantities of ether linkages are unavoidable in the polycarbonate producing reaction, and because these ether linkages are suspect in the biodegradation of polyurethanes, the quantity of macroglycol should be minimized to thereby reduce the number of ether linkages in the polycarbonate urethane. In order to maintain the total number of equivalents of hydroxyl terminal groups approximately equal to the total number of equivalents of isocyanate terminal groups, minimizing the polycarbonate soft segment necessitates proportionally increasing the chain extender hard segment in the three component polyurethane system. Therefore, the ratio of equivalents of chain extender to macroglycol should be as high as possible. A consequence of increasing this ratio (i.e., increasing the amount of chain extender with respect to macroglycol) is an increase in hardness of the polyurethane. Typically, polycarbonate urethanes of hardnesses, measured on the Shore scale, less than 70A show small amounts of biodegradation. Polycarbonate urethanes of Shore 75A and greater show virtually no biodegradation.

The ratio of equivalents of chain extender to polycarbonate and the resultant hardness is a complex function that includes the chemical nature of the components of the urethane system and their relative proportions. However, in general, the hardness is a function of the molecular weight of both chain extender segment and polycarbonate segment and the ratio of equivalents thereof. Typically, the 4,4'-methylene bisphenyl diisocyanate (MDI) based systems, a 1,4-butanediol chain extender of molecular weight 90 and a polycarbonate urethane of molecular weight of approximately 2000 will require a ratio of equivalents of at least 1.5 to 1 and no greater than 12 to 1 to provide non-biodegrading polymers. Preferably, the ratio should be at least 2 to 1 and less than 6 to 1. For a similar system using a polycarbonate glycol segment of molecular weight of about 1000, the preferred ration should be at least 1 to 1 and no greater than 3 to 1. A polycarbonate glycol having a molecular weight of about 500 would require a ratio in the range of 1.2 to 1.5:1.

The lower range of the preferred ratio of chain extender to macroglycol typically yields polyurethanes of Shore 80A hardness. The upper range of ratios typically yields polycarbonate urethanes on the order of Shore 75D. The preferred elastomeric and biostable polycarbonate urethanes for most medical devices would have a Shore hardness of approximately 85A.

Generally speaking, it is desirable to control somewhat the cross-linking that occurs during polymerization of the polycarbonate urethane polymer. A polymerized molecular weight of between 80,000 and 200,000 Daltons, for example on the order of about 120,000 Daltons (such molecular weights being determined by measurement according to the polystyrene standard), is desired so that the resultant polymer will have a viscosity at a solids content of 43% of between 900,000 and 1,800,000 centipoise, typically on the order of about 1,000,000 centipoise. Cross-linking can be controlled by avoiding an isocyanate-rich situation. Of course, the general relationship between the isocyanate groups and the total hydroxyl (and/or amine) groups of the reactants should be on the order of 1 to 1. Cross-linking can be controlled by controlling the reaction temperatures and shading the molar ratios in a direction to be certain that the reactant charge is not isocyanate-rich; alternatively a termination reactant such as ethanol can be included in order to block excess isocyanate groups which could result in cross-linking which is greater than desired.

Concerning the preparation of the polycarbonate urethane polymers, they can be reacted in a single-stage reactant charge, or they can be reacted in multiple states, preferably in two stages, with or without a catalyst and heat. Other components such as antioxidants, extrusion agents and the like can be included, although typically there would be a tendency and preference to exclude such additional components when a medical-grade polymer is being prepared.

Additionally, the polycarbonate urethane polymers can be polymerized in suitable solvents, typically polar organic solvents in order to ensure a complete and homogeneous reaction. Solvents include dimethylacetamide, dimethylformamide, dimethylsulfoxide toluene, xylene, m-pyrrol, tetrahydrofuran, cyclohexanone, 2-pyrrolidone, and the like, or combinations thereof. These solvents can also be used to delivery the polymers to the ePTFE layer of the present invention.

A particularly desirable polycarbonate urethane is the reaction product of polyhexamethylenecarbonate diol, with methylene bisphenyl diisocyanate and the chain extender 1,4-butanediol.

The use of the elastomeric bonding agent in solution is particularly beneficial in that by coating the surface 19 of ePFTE layer 14, the bonding agent solution enters the pores 18 of layer 14 defined by the IND of the ePTFE layer. As the ePTFE is a highly hydrophobic material, it is difficult to apply a bonding agent directly to the surface thereof. By providing a bonding agent which may be disposed within the micropores of the ePFTE structure, enhanced bonding attachment between the bonding agent and the ePFTE surface is achieved.

The bonding agents of the present invention, particularly the materials noted above and, more particularly, polycarbonate urethanes, such as those formed from the reaction of aliphatic macroglycols and aromatic or aliphatic diisocyanates, are elastomeric materials which exhibit elastic properties. Conventional ePTFE is generally regarded as an inelastic material, i.e., even though it can be further stretched, it has little memory. Therefore, conventional ePTFE exhibits a relatively low degree of longitudinal compliance. Also, suture holes placed in conventional ePTFE structures do not self-seal, due to the inelasticity of the ePTFE material. By applying an elastomeric coating to the ePTFE structure, both longitudinal compliance and suture hole sealing are enhanced.

In a preferred embodiment, the elastomeric boding agent may contribute to re-sealable qualities, or puncture-sealing characteristics of the composite structure. If the bonding agent is a highly elastic substance, this may impart re-sealable quantities to the composite structure. This is especially desirous in order to seal a hole created by a suture, or when the self-sealing graft may be preferably used as a vascular access device. When used as an access device, the graft allows repeated access to the blood stream through punctures, which close after removal of the penetrating member (such as, e.g., a hypodermic needle or cannula) which provided the access.

The ePTFE self-sealing graft can be used for any medical technique in which repeated hemoaccess is required, for example, but without intending to limit the possible applications, intravenous drug administration, chronic insulin injections, chemotherapy, frequent blood samples, connection to artificial lungs, and hyperalimentation. The self-sealing ePTFE graft is ideally suited for use in chronic hemodialysis access, e.g., in a looped forearm graft fistula, straight forearm graft fistula, an axillary graft fistula, or any other AV fistula application. The self-sealing capabilities of the graft are preferred to provide a graft with greater suture retention, and also to prevent excessive bleeding from a graft after puncture (whether in venous access or otherwise).

Referring again to Figure 1, textile layer 12 is secured to surface 19 of ePTFE layer 14 which has been coated with bonding agent 20. The textile layer 12 is secured by placing it in contact with the bonding agent. As it will be described in further detail hereinbelow, this process can be performed either by mechanical, chemical or thermal techniques or combinations thereof.

The composite prosthesis 10 may be used in various vascular applications in planar form as a vascular patch or in tubular form as a graft. The textile surface may be designed as a tissue contacting surface in order to promote enhanced cellular ingrowth which contributes to the long term patency of the prosthesis. The ePTFE surface 14 may be used as a blood contacting surface so as to minimize leakage and to provide a generally anti-thrombogetic surface. While this is the preferred usage of the composite prosthesis of the present invention, in certain situations, the layers may be reversed where indicated.

The present invention provides for various embodiments of composite ePTFE/textile prosthesis.

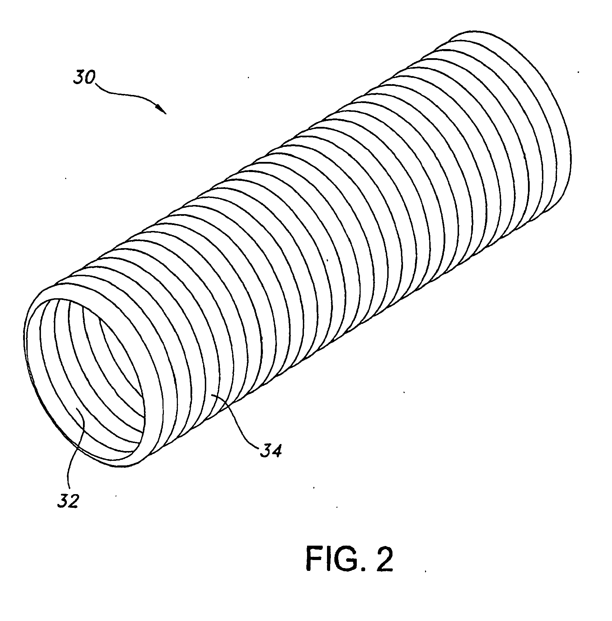

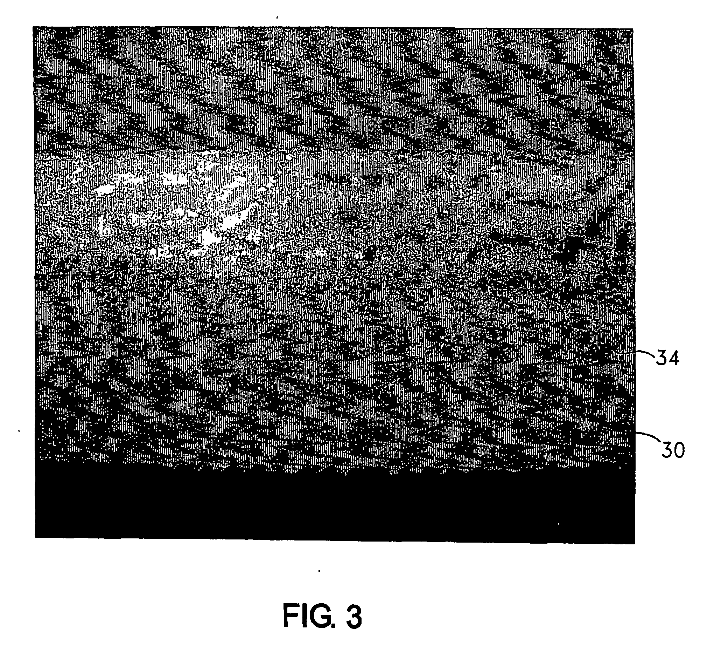

With reference to Figures 2 and 3, a ePTFE-lined textile graft 30 is shown. Graft 30 includes an elongate textile tube having opposed inner and outer surfaces 32,34. As the graft 30 of the present invention is a composite of ePTFE and textile, the textile tube may be formed thinner than is traditionally used for textile grafts. A thin-walled liner of an ePTFE tube is applied to the internal surface of the textile tube to form the composite graft. The ePTFE liner reduces the porosity of the textile tube so that the textile tube need not be coated with a hemostatic agent such as collagen which is typically impregnated into the textile structure. The overall wall thickness of composite graft 30 is thinner than an equivalent conventional textile grafts.

While the composite graft 30 of Figures 2 and 3 employs the ePTFE liner on the internal surface of the textile tube, it of course may be appreciated that the ePTFE liner may be applied to the exterior surface of the textile tube.

The composite ePTFE-lined textile graft is desirably formed as follows. A thin ePFTE tube is formed in a conventional forming process such as by tubular extrusion or by sheet extrusion where the sheet is formed into a tubular configuration. The ePTFE tube is placed over a stainless steel mandrel and the ends of the tube are secured. The ePTFE tube is then spray coated with an adhesive solution of anywhere from 1% - 15% Corethane® urethane range, 2.5 W30 in DMAc. As noted above, other adhesive solutions may also be employed. The coated ePTFE tube is placed in an oven heated in a range from 18°C to 150°C for 5 minutes to overnight to dry off the solution. If desired, the spray coating and drying process can be repeated multiple times to add more adhesive to the ePTFE tube. The coated ePTFE tube is then covered with the textile tube to form the composite prosthesis. One or more layers of elastic tubing, preferably silicone, is then placed over the composite structure. This holds the composite structure together and assures that complete contact and adequate pressure is maintained for bonding purposes. The assembly of the composite graft within the elastic tubing is placed in an oven and heated in a range of 180°C - 220°C for 5-30 minutes to bond the layers together.

Thereafter, the ePTFE lined textile graft may be crimped along the tubular surface thereof to impart longitudinal compliance, kink resistance and enhanced handling characteristics. The crimp may be provided by placing a coil of metal or plastic wire around a stainless steel mandrel. The graft 30 is slid over the mandrel and the coil wire. Another coil is wrapped around the assembly over the graft to fit between the spaces of the inner coil. The assembly is then heat set and results in the formation of the desired crimp pattern. It is further contemplated that other conventional crimping processes may also be used to impart a crimp to the ePTFE textile graft.

In order to further enhance the crush and kink resistance of the graft, the graft can be wrapped with a polypropylene monofilament. This monofilament is wrapped in a helical configuration and adhered to the outer surface of the graft either by partially melting the monofilament to the graft or by use of an adhesive.

The ePTFE-lined textile graft exhibits advantages over conventional textile grafts in that the ePTFE liner acts as a barrier membrane which results in less incidences of bleeding without the need to coat the textile graft in collagen. The wall thickness of the composite structure may be reduced while still maintaining the handling characteristics, especially where the graft is crimped. A reduction in suture hole bleeding is seen in that the elastic bonding agent used to bond the textile to the ePTFE, renders the ePTFE liner self-sealing.

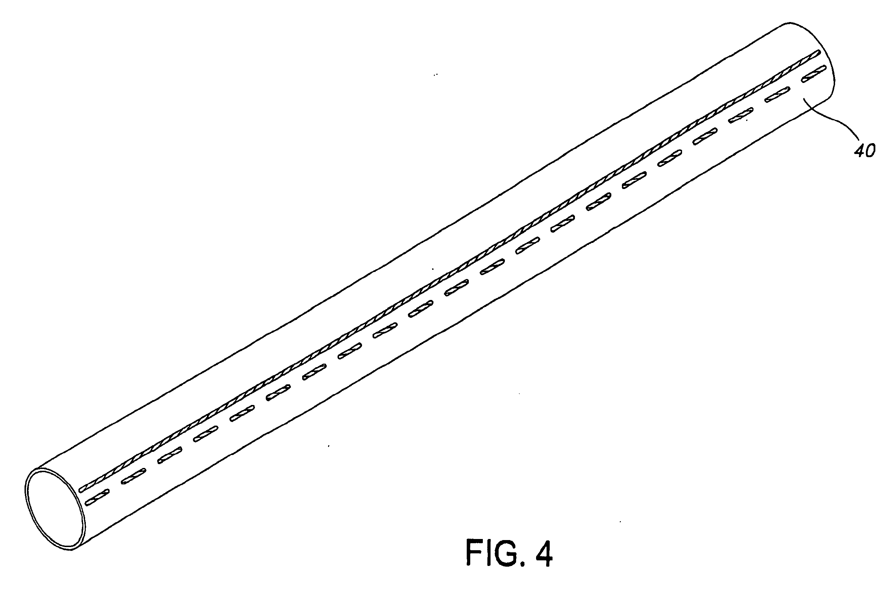

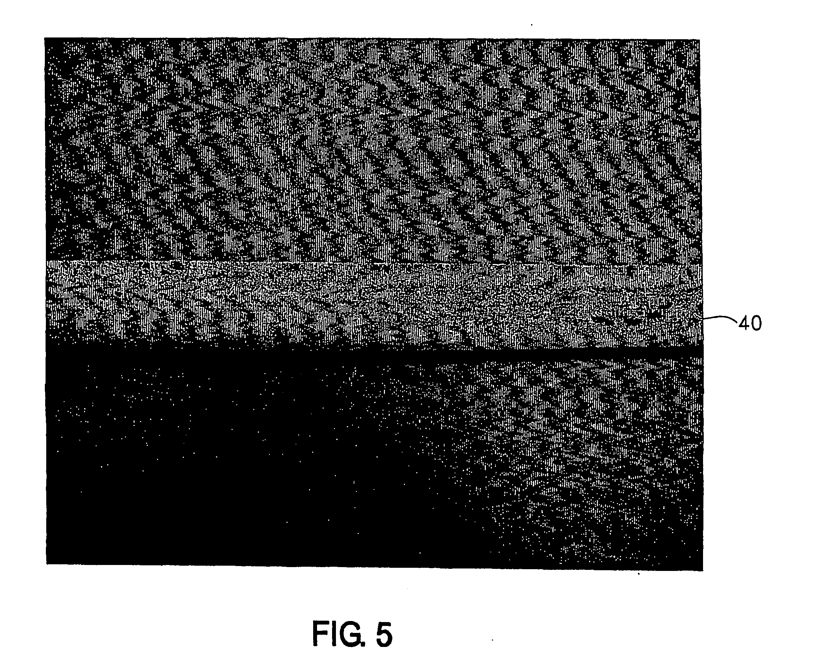

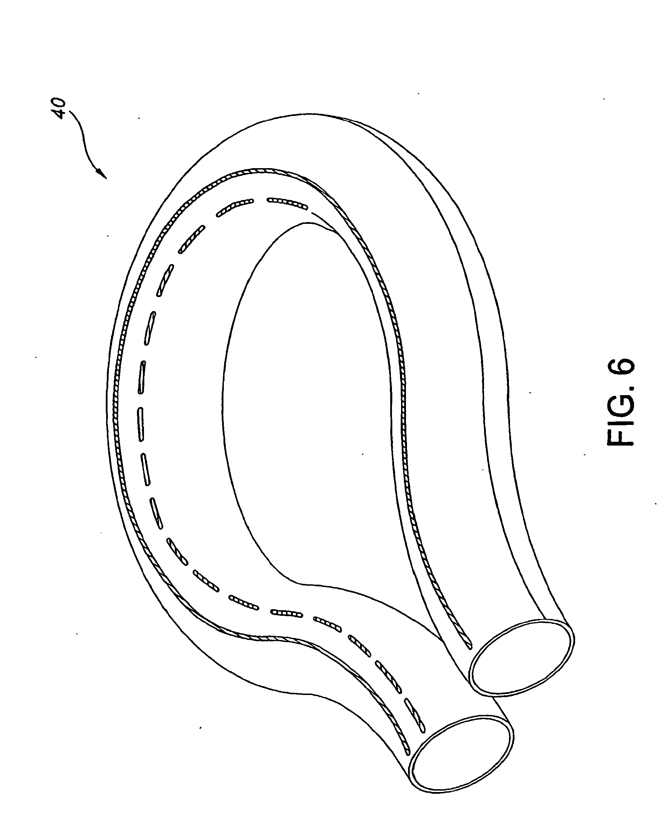

Referring now Figures 4, 5 and 6, a further embodiment of the composite ePTFE textile prosthesis of the present invention is shown. A textile covered ePTFE vascular graft 40 is shown. Graft 40 includes an elongate ePTFE tube having positioned thereover a textile tube. The ePTFE tube is bonded to the textile tube by an elastomeric bonding agent.

The process for forming the textile covered ePTFE vascular graft may be described as follows.

An ePTFE tube formed preferably by tubular paste extrusion is placed over a stainless steel mandrel. The ends of the ePTFE tube are secured. The ePTFE tube is coated using an adhesive solution of anywhere from 1% - 15% range Corethane®, 2.5 W30 and DMAc. The coated ePTFE tubular structure is then placed in an oven heated in a range from 18°C to 150°C for 5 minutes to overnight to dry off the solution. The coating and drying process can be repeated multiple times to add more adhesive to the ePTFE tubular structure.

Once dried, the ePTFE tubular structure may be longitudinally compressed in the axial direction to between 1% to 85% of its length to coil the fibrils of the ePTFE. The amount of desired compression may depend upon the amount of longitudinal expansion that was imparted to the base PTFE green tube to create the ePTFE tube. Longitudinal expansion and compression may be balanced to achieve the desired properties. This is done to enhance the longitudinal stretch properties of the resultant graft. The longitudinal compression process can be performed either by manual compression or by thermal compression.

The compressed ePTFE tube is then covered with a thin layer of the textile tube. One or more layers of elastic tubing, preferably silicone, is placed over the composite. This holds the composite together and assures that there is complete contact and adequate pressure. The assembly is then placed in a 205°C oven for approximately 10-20 minutes to bond the layers together.

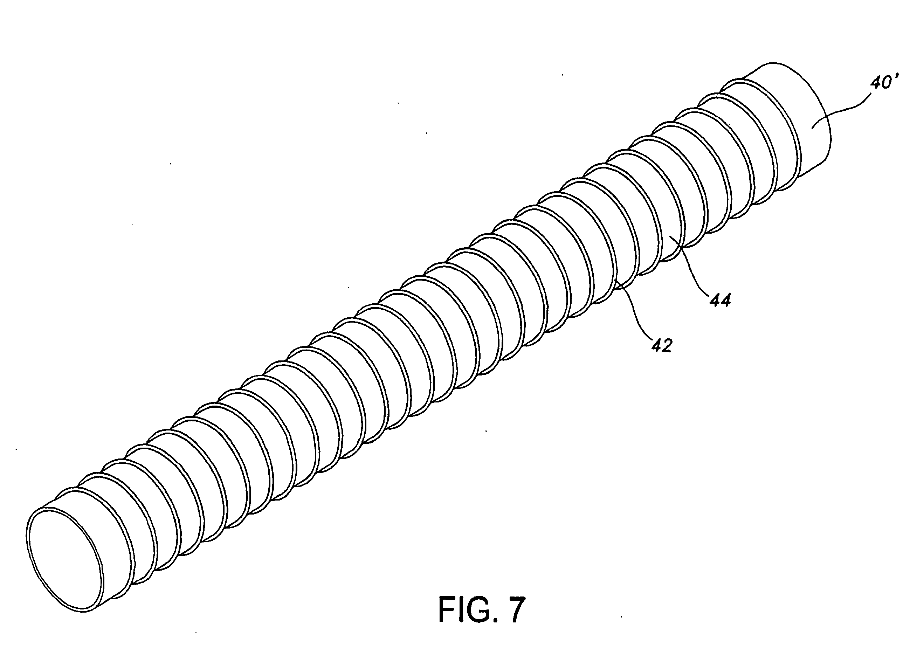

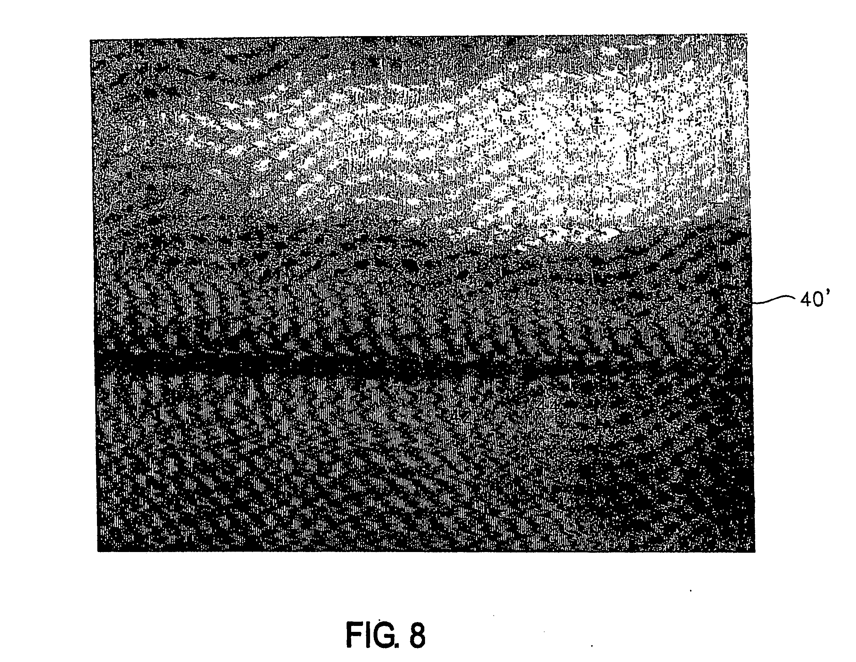

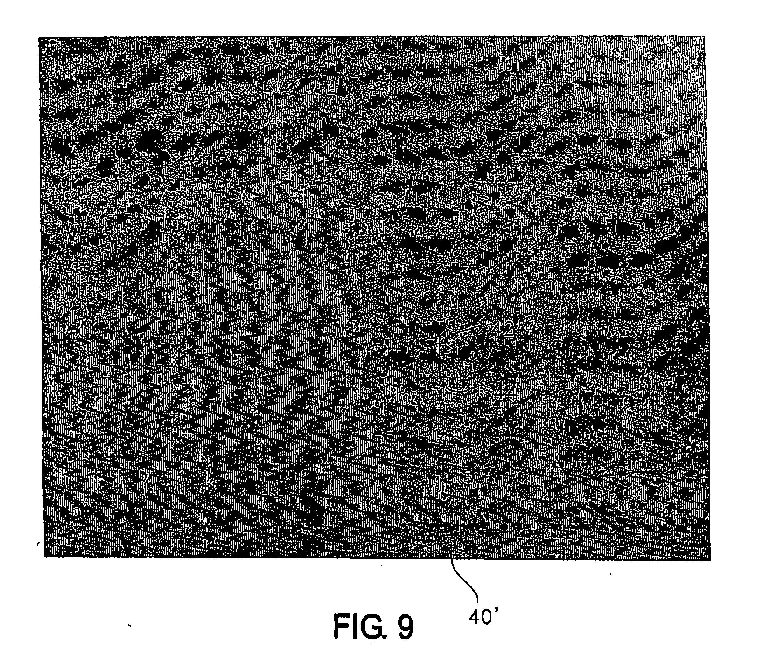

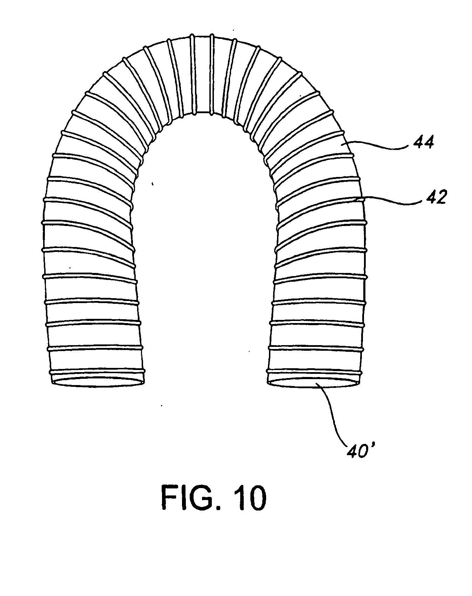

As noted above and as shown in Figures 7-10, the composite graft 40' can be wrapped with a polypropylene monofilament 42 which is adhered to the outer surface 44 by melting or use of an adhesive. The polypropylene monofilament 42 will increase the crush and kink resistance of the graft 40'. Again, the graft can be crimped in a convention manner to yield a crimped graft.

The textile covered ePTFE graft exhibits superior longitudinal strength as compared with conventional ePTFE vascular grafts. The composite structure maintains high suture retention strength and reduced suture hole bleeding. This is especially beneficial when used as a dialysis access graft in that the composite structure has increased strength and reduced puncture bleeding. This is achieved primarily by the use of an elastomeric bonding agent between the textile tubular structure and the ePTFE tubular structure in which the elastic bonding agent has a tendency to self-seal suture holes.

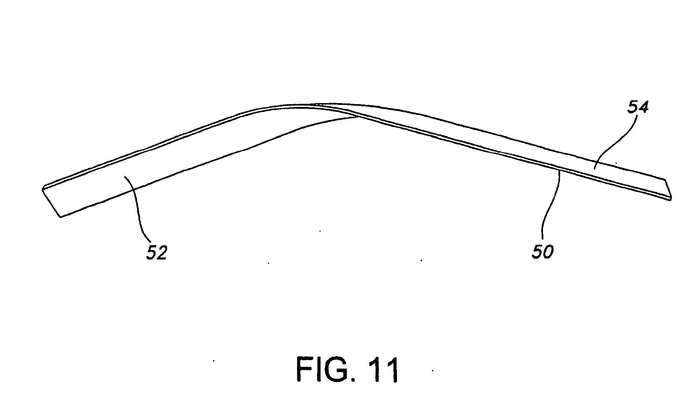

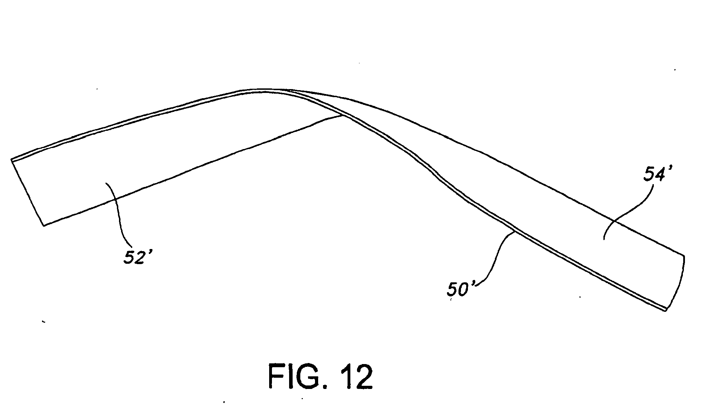

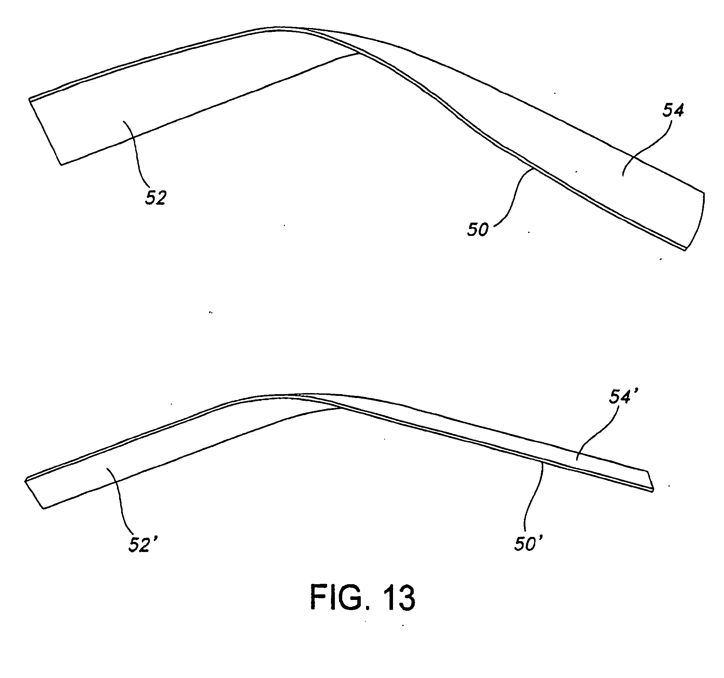

Referring now to Figures 11-13, a textile reinforced ePTFE vascular patch 50, 50' is shown. The vascular patch 50, 50' of the present invention is constructed of a thin layer of membrane of ePTFE 52, 52' which is generally in an elongate planar shape. The ePTFE membrane 52, 52'is bonded to a thin layer of textile material 54, 54' which is also formed in an elongate planar configuration. The ePTFE layer 52, 52' is bonded to the textile layer 54, 54' by use of an elastomeric bonding agent. The composite structure can be formed of a thickness less than either conventional textile or ePTFE vascular patches. This enables the patch to exhibit enhanced handling characteristics.

As is well known, the vascular patch may be used to seal an incision in the vascular wall or otherwise repair a soft tissue area in the body. The ePTFE surface of the vascular patch would be desirably used as the blood contacting side of the patch. This would provide a smooth luminal surface and would reduce thrombus formation. The textile surface is desirably opposed to the blood contacting surface so as to promote cellular ingrowth and healing.

The composite vascular patch may be formed by applying the bonding agent as above described to one surface of the ePTFE layer. Thereafter, the textile layer would be applied to the coated layer of ePTFE. The composite may be bonded by the application of heat and pressure to form the composite structure. The composite vascular patch of the present invention exhibits many of the above stated benefits of using ePTFE in combination with a textile material. The patches of the present invention may also be formed by first making a tubular construction and then cutting the requisite planar shape therefrom.

Various changes to the foregoing described and shown structures will now be evident to those skilled in the art. Accordingly, the particularly disclosed scope of the invention is set forth in the following claims.

## Claims

1. A composite multilayer implantable structure comprising:
a first layer formed of textile material;
a second layer formed of expanded polytetrafluoroethyene having a porous microstructure defined by nodes interconnected by fibrils; and
an elastomeric bonding agent applied to one of said layers and disposed within the pores of said microstructure for securing said first layer to said second layer.

2. A composite structure of claim 1 wherein said bonding agent is applied to one surface of said second layer.

3. A composite structure of claim 1 wherein said bonding agent is selected form the group consisting of urethanes, styrene/isobutylene/styrene block copolymers, silicones and combinations thereof.

4. A composite structure of claim 1 wherein said first layer comprises a textile pattern selected from the group comprising knits, weaves, stretch-knits, braids, non-woven textile structures and combinations thereof.

5. A composite structure of claim 2 wherein said first layer is placed in contact with said one surface of said second layer.

6. A composite structure of claim 1 wherein said first and second layers are substantially planar.

7. A composite structure of claim 6 wherein said first and second planar layers form a vascular patch.

8. A composite structure of claim 7 wherein said vascular patch includes said first layer being a blood contact layer and said second layer being a tissue contacting layer.

9. A composite structure of claim 1 wherein said first and second layers are substantially tubular.

10. A composite structure of claim 9 wherein said first and second tubular layers form an elongate tubular vascular graft.

11. A composite structure of claim 10 wherein said vascular graft has an inner blood-contacting second layer and an outer tissue-contacting first layer.

12. A composite structure of claim 10 wherein said vascular graft has an inner blood-contacting textile layer and an outer tissue-contacting non-textile layer.

13. A composite structure of claim 10 wherein said graft includes a plurality of longitudinally spaced crimps therealong.

14. A composite structure of claim 10 wherein said graft is helically wrapped with a monofilament externally therearound.

15. A composite structure of claim 14 wherein said monofilament comprises polypropylene.

16. A composite structure of claim 15 wherein said monofilament is attached by heat bonding.

17. A composite structure of claim 10 wherein said graft includes an external support coil helically positioned thereover.

18. A composite structure of claim 10 wherein said graft includes a support coil helically positioned between said first and second tubular layers.

19. A composite structure of claim 10 wherein said elastomeric bonding agent is self-sealing.

20. A composite structure of claim 10 wherein said composite tubular structure is longitudinally compressed.

21. A composite structure of claim 10 wherein said vascular graft has enhanced tear-resistant characteristics.

22. A composite structure of claim 1 wherein said textile material comprises PET and the elastomeric bonding agent is a polycarbonate urethane.

23. A composite structure of claim 1 wherein said composite structure further comprises a third layer.

24. A composite structure of claim 23 wherein said third layer is positioned adjacent said second layer.

25. A composite structure of claim 24 wherein said third layer is ePTFE.

26. A composite structure of claim 23 wherein said third layer is positioned said adjacent said first layer.

27. A composite structure of claim 26 wherein said third layer is formed of textile material.

28. A composite structure of claim 1 wherein said elastomeric bonding agent is applied to said second layer in solution.

29. A composite structure of claim 28 wherein said solution includes dimethylacetamide.

30. A composite structure of claim 1 wherein said bonding agent is applied by thermal processing means.

31. A method of forming a vascular prosthesis comprising the steps of:
forming an ePTFE layer having opposed surfaces comprising a microporous structure of nodes interconnected by fibrils;
forming a textile layer having opposed surfaces;
applying a coating of an elastomeric bonding agent to one of said opposed surfaces; and
securing said ePTFE and said textile layer together with said bonding agent being disposed in said microporous structure.

32. A method of claim 31 wherein said applying step includes:
applying a solution of said bonding agent.

33. A method of claim 32 wherein said applying step further includes:
spray coating said surface of said ePTFE with said solution.

34. A method of forming a textile ePTFE composite graft comprising:
providing a tubular textile structure having opposed surfaces;
providing a tubular ePTFE liner structure having opposed surfaces and having a microporous structure of nodes interconnected by fibrils;
applying a coating of an elastomeric bonding agent to one of said opposed surfaces; and
securing said textile structure and said ePTFE liner structure with said bonding agent being present in micropores of said microporous structure.

35. A method of claim 34 wherein said tubular textile structure defines an inner and outer surface.

36. A method of claim 35 wherein said ePTFE liner structure is applied to said outer textile surface.

37. A method of claim 35 wherein said ePTFE liner structure is applied to said inner textile surface.

38. A method of forming a textile covered ePTFE graft, comprising the steps of:
providing an ePTFE tube having a microporous structure of nodes interconnected by fibrils;
applying a coating of an elastomeric bonding agent to a surface of said ePTFE tube with said bonding agent being disposed within said micropores thereof; and
securing a textile tube to said coated surface of said ePTFE tube.

39. A method of forming a composite implantable patch comprising the steps of:
providing an elongate planar ePTFE substrate, said substrate having a microporous structure defined by nodes interconnected by fibrils;
applying a coating of an elastomeric bonding agent to one surface of said ePTFE substrate, said bonding agent being disposed within said micropores thereof; and
securing an elongate planar textile substrate to said coated surface of said ePTFE substrate.

## Patentansprüche

1. Eine mehrschichtige, implantierbare Verbundstruktur, die:
eine erste Schicht, die aus einem textilen Material hergestellt ist;
eine zweite Schicht, die aus einem expandierten Polytetrafluorethylen mit einer porösen Mikrostruktur, die durch Knoten definiert wird, die durch Fibrillen miteinander verbunden sind, hergestellt ist; und
ein elastomeres Klebemittel, das auf eine der Schichten aufgetragen ist und in den Poren der Mikrostruktur zur Befestigung der ersten Schicht an die zweite Schicht positioniert ist,
umfasst.

2. Eine Verbundstruktur von Anspruch 1, worin das Klebemittel auf eine Oberfläche der zweiten Schicht aufgetragen wird.

3. Eine Verbundstruktur von Anspruch 1, worin das Klebemittel aus der Gruppe ausgewählt ist, die aus Urethanen, Styrol/Isobutylen/Styrol-Blockcopolymeren, Silikonen und Kombinationen davon besteht.

4. Eine Verbundstruktur von Anspruch 1, worin die erste Schicht ein textiles Muster umfasst, das aus der Gruppe ausgewählt ist, die aus Maschenwaren, Webwaren, Dehnmaschenwaren, Borten, Fliesstextilstrukturen und Kombinationen davon besteht.

5. Eine Verbundstruktur von Anspruch 2, worin die erste Schicht mit der einen Oberfläche der zweiten Schicht in Kontakt gebracht wird.

6. Eine Verbundstruktur von Anspruch 1, worin die ersten und zweiten Schichten im Wesentlichen planar sind.

7. Eine Verbundstruktur von Anspruch 6, worin die ersten und zweiten planaren Schichten einen Gefäßflicken ausbilden.

8. Eine Verbundstruktur von Anspruch 7, worin der Gefäßflicken die erste Schicht umfasst, die eine Schicht mit Blutkontakt ist, und die zweite Schicht eine Schicht mit Gewebekontakt ist.

9. Eine Verbundstruktur von Anspruch 1, worin die ersten und zweiten Schichten im Wesentlichen röhrenförmig sind.

10. Eine Verbundstruktur von Anspruch 9, worin die ersten und zweiten röhrenförmigen Schichten ein lang gezogenes röhrenförmiges Gefäßtransplantat ausbilden.

11. Eine Verbundstruktur von Anspruch 10, worin das Gefäßtransplantat eine innere zweite Schicht mit Blutkontakt und eine äußere Schicht mit Gewebekontakt aufweist.

12. Eine Verbundstruktur von Anspruch 10, worin das Gefäßtransplantat eine innere Textilschicht mit Blutkontakt und eine äußere, nicht textile Schicht mit Gewebekontakt aufweist.

13. Eine Verbundstruktur von Anspruch 10, worin das Transplantat eine Vielzahl von Falten in Längsabstand daran entlang umfasst.

14. Eine Verbundstruktur von Anspruch 10, worin das Transplantat helikal mit einem Monofilament extern darum umwickelt ist.

15. Eine Verbundstruktur von Anspruch 14, worin das Monofilament Polypropylen umfasst.

16. Eine Verbundstruktur von Anspruch 15, worin das Monofilament durch Wärmeverkleben befestigt ist.

17. Eine Verbundstruktur von Anspruch 10, worin das Transplantat eine externe Trägerspirale umfasst, die helikal darauf positioniert ist.

18. Eine Verbundstruktur von Anspruch 10, worin das Transplantat eine Trägerspirale umfasst, die helikal zwischen den ersten und zweiten röhrenförmigen Schichten positioniert ist.

19. Eine Verbundstruktur von Anspruch 10, worin das elastomere Klebemittel selbstversiegelnd ist.

20. Eine Verbundstruktur von Anspruch 10, worin die röhrenförmige Verbundstruktur in Längsrichtung komprimiert ist.

21. Eine Verbundstruktur von Anspruch 10, worin das Gefäßtransplantat verbesserte reißwiderstandsfähige Eigenschaften aufweist.

22. Eine Verbundstruktur von Anspruch 1, worin das textile Material PET umfasst und das elastomere Klebemittel ein Polycarbonaturethan ist.

23. Eine Verbundstruktur von Anspruch 1, worin die Verbundstruktur zusätzlich eine dritte Schicht umfasst.

24. Eine Verbundstruktur von Anspruch 23, worin die dritte Schicht benachbart zu der zweiten Schicht positioniert ist.

25. Eine Verbundstruktur von Anspruch 24, worin die dritte Schicht ePTFE ist.

26. Eine Verbundstruktur von Anspruch 23, worin die dritte Schicht benachbart zu der ersten Schicht positioniert ist.

27. Eine Verbundstruktur von Anspruch 26, worin die dritte Schicht aus einem textilen Material gebildet ist.

28. Eine Verbundstruktur von Anspruch 1, worin das elastomere Klebemittel auf die zweite Schicht in Lösung aufgetragen wird.

29. Eine Verbundstruktur von Anspruch 28, worin die Lösung Dimethylacetamid umfasst.

30. Eine Verbundstruktur von Anspruch 1, worin das Klebemittel durch thermische Prozessierungsmittel aufgetragen wird.

31. Ein Verfahren zur Herstellung einer Gefäßprothese, das die Schritte:
des Ausbildens einer ePTFE-Schicht mit gegenüberliegenden Oberflächen, die eine mikroporöse Struktur aus Knoten umfasst, die durch Fibrillen miteinander verbunden sind;
des Ausbildens einer textilen Schicht mit gegenüberliegenden Oberflächen;
des Auftragens einer Beschichtung aus einem elastomeren Klebemittel auf eine der gegenüberliegenden Oberflächen; und
des Befestigens der ePTFE- und der textilen Schicht miteinander mit dem Klebemittel , das in der microporösen Struktur vorhanden ist,
umfasst.

32. Ein Verfahren von Anspruch 31, worin der Auftragungsschritt das Auftragen einer Lösung des Klebemittels umfasst.

33. Ein Verfahren von Anspruch 32, worin der Auftragungsschritt zusätzlich die Sprühbeschichtung der Oberfläche des ePTFE mit der Lösung umfasst.

34. Ein Verfahren zur Herstellung eines textilen ePTFE-Verbundtransplantats, das :
das Bereitstellen einer röhrenförmigen, textilen Struktur mit gegenüberliegenden Oberflächen;
das Bereitstellen einer röhrenförmigen ePTFE-Einlagestruktur mit gegenüberliegenden Oberflächen und mit einer mikroporösen Struktur aus Knoten, die durch Fibrillen miteinander verbunden sind;
das Auftragen einer Beschichtung aus einem elastomeren Klebemittel auf eine der gegenüberliegenden Oberflächen; und
das Befestigen der textilen Struktur und der ePTFE-Einlagestruktur mit dem Klebemittel, das in den Mikroporen der mikroporösen Struktur vorhanden ist,
umfasst.

35. Ein Verfahren von Anspruch 34, worin die röhrenförmige textile Struktur eine innere und eine äußere Oberfläche definiert.

36. Ein Verfahren von Anspruch 35, worin die ePTFE-Einlagestruktur auf die äußere textile Oberfläche aufgetragen wird.

37. Ein Verfahren von Anspruch35, worin die ePTFE-Einlagestruktur auf die innere textile Oberfläche aufgetragen wird.

38. Ein Verfahren zur Herstellung eines mit Textil bedeckten ePTFE-Transplantats, das die Schritte:
des Bereitstellens einer ePTFE-Röhre mit einer mikroporösen Struktur aus Knoten,
die durch Fibrillen miteinander verbunden sind;
des Auftragens einer Beschichtung aus einem elastomeren Klebemittel auf eine Oberfläche der ePTFE-Röhre, wobei das Klebemittel in den Microporen davon positioniert wird; und
des Befestigens einer textilen Röhre an die beschichtete Oberfläche der ePTFE-Röhre,
umfasst.

39. Ein Verfahren zur Herstellung eines implantierbaren Verbundflickens, das die Schritte:
des Bereitstellens eines lang gezogenen, planaren ePTFE-Substrats, wobei das Substrat eine mikroporöse Struktur aufweist, die durch Knoten definiert ist, die durch Fibrillen miteinander verbunden sind;
des Auftragens einer Beschichtung aus einem elastomeren Klebemittel auf eine Oberfläche des ePTFE-Substrats, wobei das Klebemittel in den Mikroporen davon positioniert ist; und
des Befestigens eines lang gezogenen, planaren Textilsubstrats an die beschichtete Oberfläche des ePTFE-Substrats,
umfasst.

## Revendications

1. Structure composite multicouche implantable comprenant :
une première couche formée de matériau textile ;
une deuxième couche formée de polytétrafluoroéthylène expansé ayant une microstructure poreuse définie par des noeuds interconnectés par des fibrilles ; et
un agent liant élastomère appliqué à l'une desdites couches et disposé à l'intérieur des pores de ladite microstructure pour fixer solidement ladite première couche à ladite deuxième couche.

2. Structure composite selon la revendication 1, dans laquelle ledit agent liant est appliqué sur une surface de ladite deuxième couche.

3. Structure composite selon la revendication 1, dans laquelle ledit agent liant est choisi dans le groupe constitué par les uréthanes, les copolymères séquencés de styrène/isobutylène/styrène, les silicones et les combinaisons de ceux-ci.

4. Structure composite selon la revendication 1, dans laquelle ladite première couche comprend un mode textile choisi dans le groupe constitué par les tricots, les tissages, les tricots extensibles, les tresses, les structures textiles non-tissées et les combinaisons de ceux-ci.

5. Structure composite selon la revendication 2, dans laquelle ladite première couche est placée en contact avec ladite surface de ladite deuxième couche.

6. Structure composite selon la revendication 1, dans laquelle lesdites première et deuxième couches sont sensiblement planes.

7. Structure composite selon la revendication 6, dans laquelle lesdites première et deuxième couches planes forment un patch vasculaire.

8. Structure composite selon la revendication 7, dans laquelle ledit patch vasculaire comprend ladite première couche qui est une couche de contact avec le sang et ladite deuxième couche qui est une couche venant en contact avec un tissu.

9. Structure composite selon la revendication 1, dans laquelle lesdites première et deuxième couches sont sensiblement tubulaires.

10. Structure composite selon la revendication 9, dans laquelle lesdites première et deuxième couches tubulaires forment un greffon vasculaire tubulaire allongé.

11. Structure composite selon la revendication 10, dans laquelle ledit greffon vasculaire comprend une deuxième couche interne venant en contact avec le sang et une première couche externe venant en contact avec un tissu.

12. Structure composite selon la revendication 10, dans laquelle ledit greffon vasculaire comprend une couche interne en textile venant en contact avec le sang et une couche externe non en textile venant en contact avec un tissu.

13. Structure composite selon la revendication 10, dans laquelle ledit greffon comprend une pluralité de frisures espacées longitudinalement le long de celui-ci.

14. Structure composite selon la revendication 10, dans laquelle ledit greffon est enveloppé hélicoïdalement avec un monofilament s'étendant extérieurement autour de celui-ci.

15. Structure composite selon la revendication 14, dans laquelle ledit monofilament comprend du polypropylène.

16. Structure composite selon la revendication 15, dans laquelle ledit monofilament est fixé par liaison thermique.

17. Structure composite selon la revendication 10, dans laquelle ledit greffon comprend un enroulement de support externe positionné hélicoïdalement par-dessus celui-ci.

18. Structure composite selon la revendication 10, dans laquelle ledit greffon comprend un enroulement de support positionné hélicoïdalement entre lesdites première et deuxième couches tubulaires.

19. Structure composite selon la revendication 10, dans laquelle ledit agent liant élastomère est autoscellant.

20. Structure composite selon la revendication 10, dans laquelle ladite structure composite tubulaire est comprimée longitudinalement.

21. Structure composite selon la revendication 10, dans laquelle ledit greffon vasculaire possède des caractéristiques renforcées de résistance à la déchirure.

22. Structure composite selon la revendication 1, dans laquelle ledit matériau textile comprend du PET et l'agent liant élastomère est un polycarbonaté uréthane.

23. Structure composite selon la revendication 1, laquelle comprend en outre une troisième couche.

24. Structure composite selon la revendication 23, dans laquelle ladite troisième couche est positionnée de façon adjacente à ladite deuxième couche.

25. Structure composite selon la revendication 24, dans laquelle ladite troisième couche est en PTFE expansé.

26. Structure composite selon la revendication 23, dans laquelle ladite troisième couche est positionnée de façon adjacente à ladite première couche.

27. Structure composite selon la revendication 26, dans laquelle ladite troisième couche est en un matériau textile.

28. Structure composite selon la revendication 1, dans laquelle ledit agent liant élastomère est appliqué sur ladite deuxième couche en solution.

29. Structure composite selon la revendication 28, dans laquelle ladite solution comprend du diméthylacétamide.

30. Structure composite selon la revendication 1, dans laquelle ledit agent liant est appliqué par des techniques de traitement thermique.

31. Méthode de formation d'une prothèse vasculaire comprenant les étapes de :
formation d'une couche de PTFE expansé ayant des surfaces opposées comprenant une structure microporeuse de noeuds interconnectés par des fibrilles ;
formation d'une couche de textile ayant des surfaces opposées ;
application d'un revêtement d'un agent liant élastomère sur l'une desdites surfaces opposées ;
et
fixation dudit PTFE expansé et de ladite couche de textile ensemble, ledit agent liant étant disposé dans ladite structure microporeuse.

32. Méthode selon la revendication 31, dans laquelle ladite étape d'application comprend :
l'application d'une solution dudit agent liant.

33. Méthode selon la revendication 32, dans laquelle ladite étape d'application comprend en outre :
le revêtement par pulvérisation de ladite surface dudit PTFE expansé avec ladite solution.

34. Méthode de formation d'un greffon composite en textile et PTFE expansé comprenant les étapes consistant à :
se pourvoir d'une structure textile tubulaire ayant des surfaces opposées ;
se pourvoir d'une structure de revêtement en PTFE expansé tubulaire ayant des surfaces opposées et ayant une structure microporeuse de noeuds interconnectés par des fibrilles ;
appliquer un revêtement d'un agent liant élastomère sur l'une desdites surfaces opposées ;
et
fixer ladite structure textile et ladite structure de revêtement en PTFE expansé avec ledit agent liant qui est présent dans des micropores de ladite structure microporeuse.

35. Méthode selon la revendication 34, dans laquelle ladite structure textile tubulaire définit une surface interne et une surface externe.

36. Méthode selon la revendication 35, dans laquelle ladite structure de revêtement en PTFE expansé est appliquée sur ladite surface textile externe.

37. Méthode selon la revendication 35, dans laquelle ladite structure de revêtement en PTFE expansé est appliquée sur ladite surface textile interne.

38. Méthode de formation d'un greffon en PTFE expansé, recouvert de textile, comprenant les étapes consistant à :
se pourvoir d'un tube en PTFE expansé ayant une structure microporeuse de noeuds interconnectés par des fibrilles ;
appliquer un revêtement d'un agent liant élastomère à une surface dudit tube en PTFE expansé, ledit agent liant étant disposé à l'intérieur desdits micropores de celui-ci ; et
fixer un tube en textile à ladite surface revêtue dudit tube en PTFE expansé.

39. Méthode de formation d'un patch composite implantable comprenant les étapes consistant à :
se pourvoir d'un substrat en PTFE expansé plan allongé,
ledit substrat ayant une structure microporeuse définie par des noeuds interconnectés par des fibrilles ;
appliquer un revêtement d'un agent liant élastomère à une surface dudit substrat en PTFE expansé, ledit agent liant étant disposé à l'intérieur desdits micropores de celui-ci ; et
fixer un substrat en textile plan allongé à ladite surface revêtue dudit substrat en PTFE expansé.
